# EUROPEAN PATENT SPECIFICATION

(11) **EP 1 765 220 B1**
(45) Date of publication and mention of the grant of the patent: **30.11.2011**
(21) Application number: 05753133.7
(22) Date of filing: 09.06.2005
(51) Int. Cl.: A61F 2/06, A61L 31/00, A61L 27/00

(54) **IMPLANTABLE DEVICE FOR DRUG DELIVERY AND IMPROVED VISIBILITY**
IMPLANTIERBARE VORRICHTUNG ZUR ARZNEIMITTELABGABE UND FÜR VERBESSERTE SICHTBARKEIT
DISPOSITIF IMPLANTABLE POUR L'ADMINISTRATION D'UN MÉDICAMENT AVEC VISIBILITÉ AMÉLIORÉE

(30) Priority: 09.06.2004 EP 04013671
(43) Date of publication of application: 28.03.2007
(73) Proprietor: J.A.C.C. GmbH, 81679 München (DE)
(72) Inventor: FROHWITTER, Bernhard, 81679 München (DE)
(74) Representative: Tomlinson, Edward James
(86) International application number: PCT/EP2005/006223
(87) International publication number: WO 2005/120393

(56) References cited:
- EP-A- 0 604 022
- EP-A- 0 761 251
- EP-A- 1 234 587
- EP-A- 1 277 449
- WO-A-02/26281
- WO-A-98/36784
- WO-A-02/060506
- WO-A-03/015664
- WO-A-03/087443
- DE-U1- 20 200 220
- US-A- 5 290 271
- US-A- 6 071 305
- US-A1- 2001 044 650
- US-A1- 2002 038 145
- US-A1- 2003 088 307
- US-A1- 2003 121 148
- US-A1- 2003 149 475
- US-A1- 2004 106 988
- US-B1- 6 645 241

## Description

### TECHNICAL FIELD

The present invention generally relates to implantable devices and therapeutic methods involving intravenous or surgical introduction of devices that are implantable into a patient, either human or non-human, such as the introduction into a region of the body such as a passage or blood vessel or other lumen or directly into soft tissue or bone.

### BACKGROUND OF THE INVENTION

In the past, various medical devices have been developed to treat a variety of medical conditions by introducing an implantable medical device partly or completely into the vascular system, esophagus, trachea, colon, biliary tract, urinary tract, pancreas, uterus or other location within a human or animal patient.

As an example, many treatments of the vascular system typically comprise the introduction of a device such as stents, catheters, cannulae, balloons or the like. Unfortunately, however, when such a device is introduced through the vascular system until positioned at the desired location, the blood vessel walls can be disturbed or even injured. Thrombosis often results at the injured site thereby causing stenosis/occlusion of the blood vessel.

Furthermore, if the medical device is positioned within the lumen of a body portion for an extended period of time, a thrombus often forms on the device itself, again causing stenosis/occlusion. As a result, the patient is placed at risk of a variety of complications, including stroke, heart attack and pulmonary embolism.

Another reason why which blood vessels undergo stenosis is through disease. For example, the most common reason causing stenosis is atherosclerosis. Atherosclerosis is a process in which deposits of fatty substances, cholesterol, cellular waste products, calcium and other substances build up in the inner lining of an artery. Plaques can grow large enough to significantly reduce the blood's flow through an artery (ischemia) The most dangerous damage, however, occurs when they become fragile and rupture. Plaques that rupture cause blood clots to form that can block blood flow or break off and can cause a heart attack or stroke.

Many medical devices and therapeutic methods are known for the treatment of atherosclerotic disease. Probably the most common one is percutaneous transluminal angioplasty (PTA). PTA is based on Gruntzig's original concept of using a noncompliant balloon mounted on a double lumen catheter. One lumen allows the balloon catheter to be advanced over a guide wire and permits injection of contrast material, while the second lumen serves to inflate the balloon to a predetermined diameter. Briefly, a balloon-tipped catheter is inserted in a patient's artery, the balloon being deflated. The tip of the catheter is then advanced to the site of the atherosclerotic plaque to be dilated. The balloon is placed within/across the stenotic segment and subsequently inflated. As a result, the balloon "cracks" the atherosclerotic plaque and expands the vessel, thereby relieving the stenosis.

A device such as an intravascular stent can be a useful adjunct to PTA, particularly in the case of either acute or threatened closure after angioplasty. The use of PTA is, however, hampered by the observation that the treated blood vessel may suffer acute occlusion immediately after or within the initial hours after the dilation procedure. Another limitation encountered in PTA is called restenosis, i.e. re-narrowing of an artery after an initial successful angioplasty. This conditions arises typically during the first six months after angioplasty and is believed to be caused by proliferation and migration of vascular endothelial cells and/or by remodelling of the arterial wall.

Nevertheless, local sustained-release delivery systems may still offer the best way to treat certain medical conditions where high local concentrations and/or controlled delivery of the therapeutic agent is desired such that problems of systemic toxicity are essentially avoided. Conditions and diseases other than atherosclerosis are treatable with stents, catheters, cannulae and other devices partly or completely inserted into the vascular system, esophagus, trachea, colon, biliary tract, urinary tract, pancreas, uterus or other location within a body portion such as a passage, lumen or blood vessel of a human or veterinary patient.

It would be desirable to develop devices and methods for reliably delivering suitable therapeutic agents directly into a body portion during or following a medical procedure, so as to treat or prevent such conditions and diseases.

Metallic stents covered with a first composite layer of a polymer and of a therapeutically active substance coated with a second layer of fibrin are disclosed in Patent Application EP-A-0 701 802.

Known stent designs further include drug-impregnated polymer-coated metallic stents and biodegradable drug-eluting polymer stents coated with paclitaxel for the treatment of atherosclerosis (EP 0 711 158 B1).

The invention of EP 0 747 069 is directed to implantable medical devices whereby the surface of the structure or at least in one part of the structure such as wells, holes, grooves, slots or the like are loaded with the therapeutic agent. An additional porous layer enables controlled release of the therapeutic agent.

EP 0 809 515 relates inter alia to a biodegradable stent with the therapeutic agent impregnated therein, i.e. in the stent material, and which is further coated with a biodegradable coating or with a porous or permeable non-biodegradable coating comprising a sustained release-dosage form of a therapeutic agent. This embodiment of the invention can provide a differential release rate of the therapeutic agent, i.e., there would be a faster release of the therapeutic agent from the coating followed by delayed release of the therapeutic agent that is impregnated in the stent matrix upon degradation of the stent matrix.

US 6,562,065 discloses an expandable medical device comprising a plurality of elongated beams, the plurality of elongated beams joined together to form a substantially cylindrical device wherein the elongated beams include a plurality of holes for containing a beneficial agent.

EP 1 277 449 A1 describes expandable stents having well-like recesses in elements forming a reticular structure which are filled with an active agent. The active agent may have a concentration profile which varies along the length of the stent.

US 2001/0044650 discloses a stent according to the introductory portion of claim 1.

### SUMMARY OF THE INVENTION

The present invention provides a stent according to claim 1. Further preferred aspects of the invention are provided according to the dependent claims.

### BRIEF DESCRIPTION OF THE DRAWINGS

The invention will now be described, by way of example only, with reference to the drawings in which:
- Fig.1: shows a cross section of a coated stent;
- Fig. 2: shows a schematic illustration of the coated stent of Fig. 1;
- Fig 2a: shows a cross section of a self expanding coated stent;
- Fig. 3: shows a cross-section of a stent having two coating layers;
- Fig. 4: shows a schematic illustration of a stent having island coating regions;
- Fig. 5: is a schematic illustration of a multiregion stent;
- Fig. 6: is a further schematic illustration of a multiregion stent; and
- Fig. 7: is a schematic illustration of a multiregion stent having an intermediate structure.

### DESCRIPTION OF THE PREFERRED EMBODIMENTS

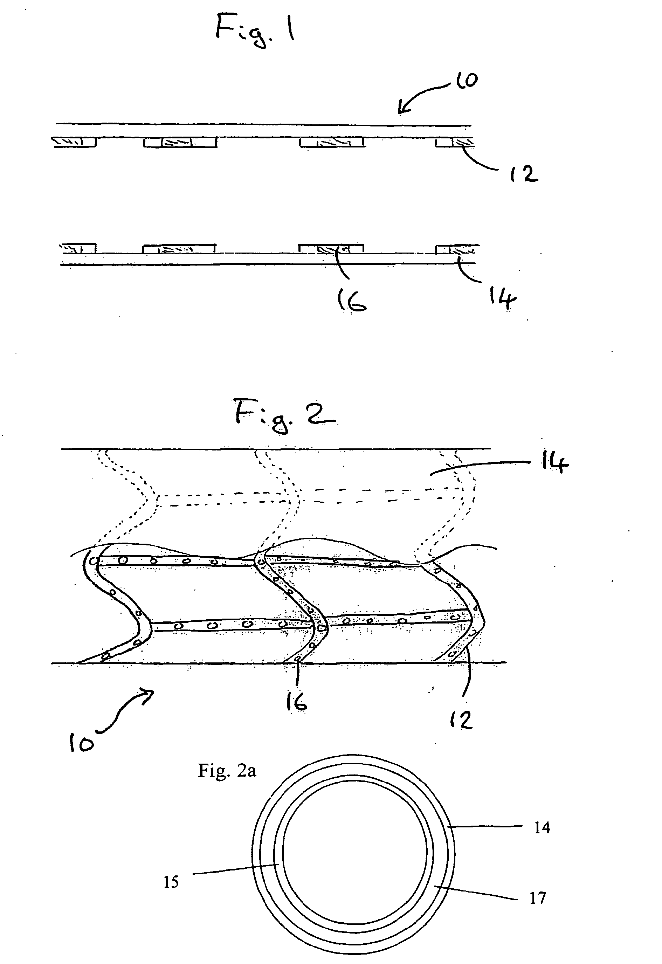

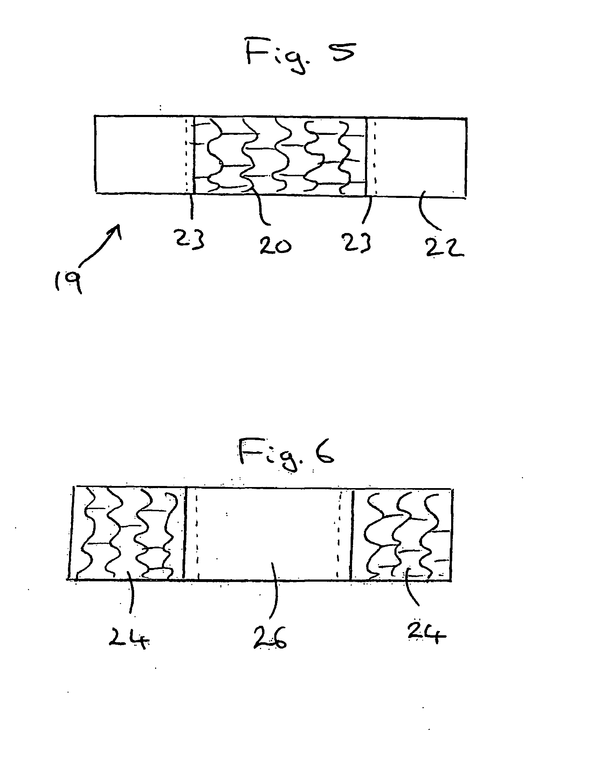

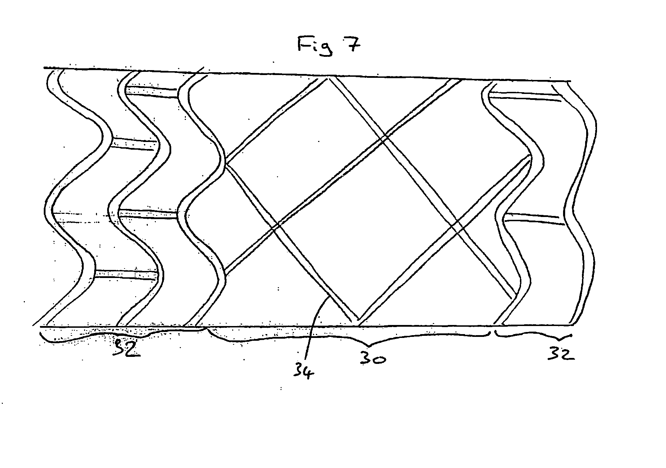

Figures 1-5 illustrate stent structures which are not part of the invention but are included to aid the understanding of the invention shown in Figures 6 and 7.

Referring to Fig. 1, there is shown a cross-section of a stent 10. The stent 10 comprises a metallic scaffolding formed of an arrangement of stainless steel struts 12 coated with a polymer layer 14. The struts 12 include therein through-holes 16 which are filled with a drug-eluting biodegradable polymer reservoirs fabricated in a manner as described in EP 0 747 069. The polymer layer 14 is also biodegradable and drug-eluting.

The stent 10 shown in Fig. 1 is shown in schematic form in Fig. 2 in which for ease of understanding the polymer layer 14 is shown only partially to reveal the scaffolding structure underneath of the struts 12 including polymer filled through-holes 16. The details of the scaffolding structure are not important for the understanding of the invention. As shown, the scaffolding is similar to the arrangement illustrated in EP-A-0 540 290 but could take other forms. Suitable stent arrangements are described in the "Handbook of Coronary Stents", second edition, Rotterdam Thoraxcenter Group, 1998, Mosby. Although the polymer layer 14 is shown as being a continuous covering, in practice, the layer could cover only the underlying scaffolding, either wholly or partly. The polymer may be the same as that filling the through holes 16, in which case a bond to these regions would generally be formed, or a different polymer may be used to provide a particular drug-elution rate profile.

If the polymer layer 14 is to provide a continuous surface such as that shown in Fig. 2, this could be obtained by attaching a sheet of polymer to the surface of the drug impregnated stent by wrapping the stent in the sheet and joining two sides of the sheet together. By exerting pressure on the sheet, at a slightly elevated temperature, a bond will form with the polymer within the through holes 16. If only the underlying scaffolding is to be covered, this could be achieved by dipping the drug in a polymer/drug solution so as to coat the exposed metal with the polymer. The solvent is then evaporated to leave the drug containing polymer coating.

For certain applications, in which a metal stent is covered with a biodegradable outer covering, it would be beneficial if the metal stent could be arranged to expand after the covering has been absorbed into the body in order to provide an optimal support of the lumen wall. If the metal stent is of the self expanding type, the biodegradable covering would have the effect of restraining the expansion of the stent within the patient. If the expansion is, however, balloon in a manner to cause plastic deformation of the outer covering within the patients lumen, this will enable the inner self-expanding stent to be inserted into position within the patient and once the degradable covering has been absorbed, a continued effective support will be achieved. A further mechanism of enabling a long term good supporting action once the degradable coating has been withdrawn would be to provide a plating on the inside of the stent of an oxide forming material, such as magnesium. As the magnesium oxidizes, the internal stresses resulting will cause the overlying metal stent to expand slightly, compensating for the absorption of the biodegradable covering. The inner surface of the stent may also comprise a biodegradable drug-eluting coating. Such an arrangement is shown in Fig. 2a in which a metal self expanding stent17 is covered with a biodegradable drug-eluting coating 14. On the inner surface of the stent 17, a further biodegradable drug eluting coating 15 is provided. The drugs eluted from the coatings 14 and 15 may be the same or may be different. The stent 17 may also be drug eluting.

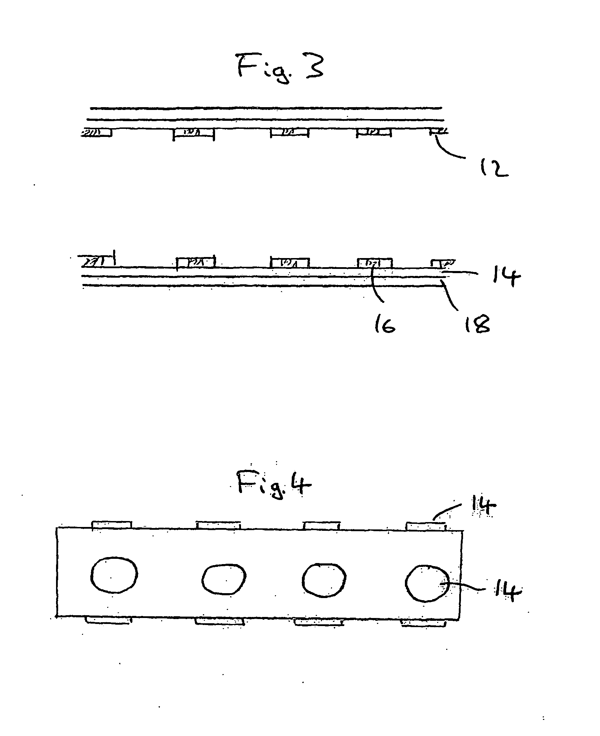

Fig. 3 shows a similar arrangement except that the stent includes two polymer layers 14 and 18 containing different therapeutic compositions. As shown in Fig. 4, the polymer layers need not be continuous but may be in the form of discrete islands on the underlying metal scaffolding.

A further aspect of the invention is illustrated in Fig. 5. This figure shows a stent 19 comprised of a central metallic region 20 and two polymer end regions 22. The metallic region 20 has a conventional scaffolding arrangement with drug eluting reservoirs therein. The polymer end regions are also drug eluting but are biodegradable. There is a region of overlap 23 in which the polymer end regions 23 overlap the underlying metallic region. In this overlap region, there are through-holes through the metal which are filled with the polymer forming the end region 22, providing a strong bond between the two regions. Accordingly, after insertion into a patient, a locally high dose of the therapeutic medicament will be provided in the region of the desired treatment and after the polymer has been adsorbed into the patient, only the relatively small region of the metallic stent would remain. This arrangement thus enables a relatively higher concentration of medicament to be provided in the desired locality than would be provided by the metallic part of the stent alone.

An embodiment of the invention is shown in Fig. 6. In this arrangement, there is a central biodegradable polymer region 26 and two metallic end regions 24. If desired, the central polymer region 26 can be formed over a metal support, as shown in Fig. 7. In this figure, a central region 30 has a metallic structure formed of struts 34 connecting to end regions 32. During manufacture, a drug-eluting polymer is cast over the struts 34 to form the polymer region 26 (not shown in Fig. 7). The struts 34 serve to provide a physical permanent connection between the end regions 32 but have a structure which is significantly more open than the metallic end region structure. Accordingly, after insertion into a patient, the central region is significantly more transparent to X-rays, for example coming from a CT scanner. As the reader will appreciate, the central region illustrated in Fig. 7 is not drawn to scale.

If, for example, the implantable device is a stent comprising three tubular sections, a central metal tubular section and a biodegradable tubular section attached at each end, this could be manufactured by forming the central metal region in a conventional fashion known to those skilled in the art of stent manufacture to provide a scaffolding structure. This could then be loaded with a drug-eluting agent, for example by filling apertures in the metal tube with a drug containing biodegradable polymer. Drug loaded biodegradable polymer tubular sections could then be attached to this metal portion using a body-compatible adhesive such as a silicone adhesive. The combined structure can then receive a full or partial coating of a biodegradable, drug eluting polymer.

Alternatively, the biodegradable polymer section could be attached to the metal portion by directly molding the polymer onto an end region of the metal. In this embodiment, the end portion of the metal region would preferably include holes drilled through the metal such that in the molding process, the polymer could enter the holes and once solidified form an attachment mechanism.

Of course, the stent could have a configuration of a central biodegradable region with non-degradable end regions attached thereto. In such an arrangement, it would be preferable for the central region to include a minimal metallic support structure to maintain the two metallic end regions in a given spatial relationship. Such an arrangement could be fabricated by cutting a pattern from a stainless steel tube such that a central region has a much lower metallic area compared with two end regions. The central region or the central region and the end regions could then receive a polymer molding.

Although in Figs 5 and 6, each polymer region is shown as being continuous, it may be desirable to provide a structure in which the polymer region is in the form of windows in the wall of an otherwise conventional metal stent. In the region of the windows, the scaffolding effect normally provided by metal struts is provided by the polymer. In such an arrangement, since the metal scaffolding is effectively continuous, there would be no requirement to have metal struts in the polymer reigon although this may be desirable to provide a desirable location and restraining capability to hold the polymer regions in place.

In addition to a stent having a metal central region and one or more polymer biodegradable end regions, a stent could be envisaged in which all regions are metallic. For example, a central region could be fabricated from stainless steel to provide long term support whilst one or both end regions could have attached thereto a stent region fabricated from a biodegradable metal alloy such as a magnesium alloy or other adsorbable metal as described in EP-A-0 966 979. Although the bonding of dissimilar metals is potentially complicated, techniques do exist, for example vacuum welding (especially electron beam welding) or gluing. It may be beneficial to include a plating layer on the stainless steel region to improve compatibility with the biodegradable metal region which could be dimensioned to fit over or within the stainless steel region. Diffusion bonding under pressure could provide a suitable joining mechanism. Again, the metal regions could include drug eluting polymer filled reservoirs and the whole or part of the structure could be coated with a drug-eluting polymer.

Recently, the use of nanofibers has been suggested for the release of NO in a controlled manner to tissues and organs, for example in WO 01/26702. Such nanofibers as described therein could be incorporated into the devices of the present invention. As an example, the fibers could be used to weave a fabric sleeve which could cover the stent structure, possible together with a biodegradable polymer matrix. Alternatively, a sleeve of such woven fibers could be used to connect to end metallic end regions. Additionally, short nanofiber lengths could be mixed with a polymer solution and the resulting mixture used to form a polymer/fibre composite structure.

Although the preceding description has concentrated on the drug-eluting possibilities for device construction, the ability to combine different materials in a single structure can also provide further benefits. One problem with existing stent designs is that when inserted into a patient, it becomes difficult to monitor the vessel in the region of the stent because the observation signal generated by the stent material is too high. Accordingly, it would be desirable to have a stent structure with a greater transparency for use in NMR or CT scanners. Such a structure can be obtained by filling voids in a relatively open stainless steel stent structure with a biodegradable polymer. After insertion into the patient, the polymer helps to support the vessel wall whilst at the same time allowing the physician to monitor the position of the stent and its local effect using conventional scanning technology. Later, as the support requirement reduces, the polymer can biodegrade. If this decrease in support capability is undesirable, a non-biodegradable polymer could be used. Such an arrangement would be similar to that shown in Figs. 5-7 but without any requirement for the polymer regions to be drug-eluting although of course this may be desirable.

## Claims

1. A stent for implantation in a lumen of a patient, said stent comprising a plurality of first supporting regions (24, 32) having metallic lumen wall supporting means and at least one second supporting region (26, 30) within or between the metallic supporting regions, the second supporting region comprising a structural polymeric lumen wall supporting means, **characterized in that** the at least one second supporting region comprises an arrangement of metal struts (34) joined to the metallic, lumen wall supporting means, the metal struts (34) of the polymeric supporting region having a ratio of projected surface area per unit surface area of the stent substantially less than a ratio of projected surface area to unit surface area of the stent of the metallic lumen wall supporting means.

2. A stent according to claim 1, wherein the structural polymer is biodegradable.

3. A stent according to claim 1 or 2 wherein the structural polymer is drug-eluting.

4. A stent according to any one of claims 1 to 3 wherein the metal lumen wall supporting means are drug eluting.

5. The stent according to claim 1, wherein the second supporting region (26, 30) is more transparent to X-rays than the first supporting regions (24, 32).

## Patentansprüche

1. Stent zur Implantation in ein Lumen eines Patienten, wobei der Stent eine Vielzahl von ersten Stützregionen (24, 32) mit metallischen Lumenwandstützmitteln und zumindest eine zweite Stützregion (26, 30) in oder zwischen den metallischen Stützregionen umfasst, wobei die zweite Stützregion ein strukturelles Polymer-Lumenwandstützmittel umfasst, **dadurch gekennzeichnet, dass** die zumindest eine zweite Stützregion eine Anordnung von Metallstreben (34) umfasst, die mit dem metallischen Lumenwandstützmittel verbunden sind, wobei die Metallstreben (34) der Polymer-Stützregion ein Verhältnis von projizierter Oberfläche pro Stentflächeneinheit aufweist, das im Wesentlichen kleiner ist als ein Verhältnis von projizierter Oberfläche zur Stentfläche des metallischen Lumenwandstützmittels.

2. Stent nach Anspruch 1, worin das strukturelle Polymer biologisch abbaubar ist.

3. Stent nach Anspruch 1 oder 2, worin das strukturelle Polymer ein Arzneimittel freisetzt.

4. Stent nach einem der Ansprüche 1 bis 3, worin das metallische Lumenwandstützmittel ein Arzneimittel freisetzt.

5. Stent nach Anspruch 1, worin die zweite Stützregion (26, 30) für Röntgenstrahlen transparenter ist als die ersten Stützregionen (24, 32).

## Revendications

1. Stent destiné à être implanté dans une lumière d'un patient, ledit stent comportant une pluralité de premières zones d'appui (24, 32) avec des moyens d'appui de paroi luminale et au moins une deuxième zone d'appui (26, 30) dans ou entre les zones d'appui métalliques, la deuxième zone d'appui comportant un moyen d'appui de paroi luminale en polymère structurel, **caractérisé en ce que** ladite au moins une deuxième zone d'appui comporte un agencement d'entretoises en métal (34) assemblées aux moyens d'appui métalliques de paroi luminale, les entretoises en métal (34) de la zone d'appui en polymère ayant un rapport surface de projection sur unité de surface du stent sensiblement inférieur à un rapport surface de projection sur unité de surface du stent des moyens d'appui métalliques de paroi luminale.

2. Stent selon la revendication 1 dans lequel le polymère structurel est biodégradable.

3. Stent selon la revendication 1 ou 2 dans lequel le polymère structurel est à élution de médicament.

4. Stent selon l'une quelconque des revendications 1 à 3 dans lequel les moyens d'appui métalliques de paroi luminale sont à élution de médicament.

5. Stent selon la revendication 1 dans lequel la deuxième zone d'appui (26, 30) est plus transparente aux rayons X que les premières zones d'appui (24, 32).
